# EUROPEAN PATENT APPLICATION

(11) **EP 3 124 590 A1**
(43) Date of publication of application: **01.02.2017**
(21) Application number: 15770254.9
(22) Date of filing: 25.03.2015
(51) Int. Cl.: C12M 1/00, B01D 29/01, B01D 35/02, B01D 35/30, C12N 1/00, C12N 5/09, C12Q 1/06

(54) **CELL CAPTURE DEVICE, CELL CAPTURE FILTER, CELL CAPTURE APPARATUS, AND METHOD FOR MANUFACTURING CELL CAPTURE DEVICE**

(30) Priority: 27.03.2014 JP 2014066515
(71) Applicant: Hitachi Chemical Co., Ltd., Chiyoda-ku Tokyo 100-6606 (JP)
(72) Inventor: KIKUHARA Yoshihito, Tokyo 100-6606 (JP); ODAGIRI Taihei, Tokyo 100-6606 (JP); KOTATO Akio, Tokyo 100-6606 (JP); KANETOMO Masafumi, Tokyo 168-0082 (JP)
(74) Representative: Hoffmann Eitle
(86) International application number: PCT/JP2015/059208
(87) International publication number: WO 2015/147086

(57) **Abstract**

Cells in a test liquid are captured and observed easily and accurately. A cell capture filter 30 is fixed by a first protrusion part 125 of a lid member 100 and a second protrusion part 225 of a housing member 200 in a cell capture device 1, and a first fitting part 130 and a second fitting part 230 are fitted with each other. This allows the cell capture filter 30 to be fixed between the lid member 100 and the housing member 200 in a state where a filter region of the cell capture filter 30 is surrounded by the looped first protrusion part 125 and the looped second protrusion part 225.

## Description

### Technical Field

The present invention relates to a cell capture device and a cell capture filter to capture cells contained in a cell dispersion, a cell capture apparatus using this cell capture device, and a method for manufacturing the cell capture device.

### Background Art

Cancer ranks high in the cause of death in the world. In Japan, more than 300,000 people per year die of cancer, and thus the early detection and the treatment of cancer are desired. In most cases, the death of humans due to cancer is caused by metastatic recurrence of cancer. The metastatic recurrence of cancer is caused by cancer cells that are from a primary lesion through blood vessels or lymph vessels and settle in and infiltrates to the blood vessel wall of another organ tissue to form micrometastases. The cancer cell circulating in a human body through blood vessels or lymph vessels as described above is called Circulating Tumor Cell (hereinafter, may be called "CTC").

In blood, many blood cell components such as red blood cells, white blood cells, and platelets are included, and the number thereof is said to be 3.5 × 10⁹ to 9 × 10⁹ pieces in 1mL of blood. On the contrary, only a several number of CTCs exist, and thus the blood is needed to be filtered in order to efficiently capture and detect CTCs from the blood cell components. Various studies have been made on such a method. For example, Patent Literature 1 has disclosed a configuration in which a ring part is provided in order to clamp and fix a filter for filtering blood and the projection part of a lid member protrudes from the pressure-contact surface of a flange to the recess part of a housing member. Patent Literature 2 has disclosed a microfluidic device provided with a polydimethylsiloxane (PDMS) upper member provided with a sample supply openings located at the upper part and the lower part of a nickel substrate having fine through-holes in the nickel substrate and a lower member provided with a sample discharge opening.

### Citation List

### Patent Literature

Patent Literature 1: Japanese Patent No. 4531310
Patent Literature 2: Japanese Unexamined Patent Publication No. 2011-163830

### Summary of Invention

### Technical Problem

In the case of the device incorporating the filter described in Patent Literature 1, however, the assembly work may be troublesome. In order to observe the filter capturing CTCs, the device needs to be disassembled. Consequently, in the processes of disassembling the device and taking out the filter, substances from the external environment may adhere to the filter and thus the accuracy of the observation result may deteriorate. When the possibility of adhesion of various pathogenic bacteria or viruses to the inside of the device is taken into account, it is considered that the disassembly process of the device to which the blood adheres forces operators to bear the burden in terms of safety. Additionally, the structure disclosed in Patent Literature 2 has large fluctuation in the height of the filter surface and thus the workability of the microscope observation of CTCs may deteriorate.

The present invention has been made in view of the above problems and a purpose of the present invention is to provide a cell capture device, a cell capture filter, and a cell capture apparatus that can capture and observe cells easily and accurately and can also reduce the fluctuation in the height of the filter surface, and a method for manufacturing the cell capture device.

### Solution to Problem

In order to achieve the purpose, a cell capture device according to one embodiment of the present invention includes: a chassis including a lid member having an introduction channel configured to introduce a test liquid into an inside, and a housing member having a discharge channel configured to discharge the test liquid to an outside; and a cell capture filter disposed on a channel inside the chassis between the introduction channel and the discharge channel, including a filter region therein provided with a through-hole in a thickness direction and configured to pass the test liquid, in which
the lid member includes a looped first protrusion part provided at a position outside of the filter region and inside of an outer edge of the cell capture filter in an assembled state and protruding outward from a surface of a side to which the cell capture filter is attached,
the housing member includes a looped second protrusion part provided at a position corresponding to the first protrusion part in the assembled state and located at a position corresponding to the first protrusion part to protrude, and the channel inside the chassis is formed by locating the first protrusion part and the second protrusion part at the corresponding position to sandwich the cell capture filter in the assembled state.

A cell capture filter according to one embodiment of the present invention includes a filter region therein provided with a through-hole in a thickness direction and configured to pass a test liquid, in which the cell capture filter is fixed without being folded, by a lid member having an introduction channel configured to introduce the test liquid into an inside and including a looped first protrusion part protruding outward from a surface of a side to which the cell capture filter is attached and a housing member having a discharge channel configured to discharge the test liquid to an outside and including a looped second protrusion part protruding outward from a surface of a side to which the cell capture filter is attached.

A cell capture filter according to one embodiment of the present invention is configured to be fixed by a lid member having an introduction channel configured to introduce a test liquid into an inside and a housing member having a discharge channel configured to discharge the test liquid to an outside, in which the lid member provides a looped first protrusion part provided at a position outside of a filter region of the cell capture filter where through-holes configured to pass the test liquid are formed in a thickness direction and inside of an outer edge of the cell capture filter in a fixed state and protruding outward from a surface of a side to which the cell capture filter is attached, the housing member provides a looped second protrusion part provided at a position corresponding to the first protrusion part in the fixed state and located at a position corresponding to the first protrusion part to protrude, the first protrusion part and the second protrusion part are located at the corresponding position to sandwich the cell capture filter in the fixed state, and the outer edge of the cell capture filter is located outside of a larger outer periphery of either the first protrusion part or the second protrusion part.

A method for manufacturing a cell capture device according to one embodiment of the present invention including a chassis having a lid member having an introduction channel configured to introduce a test liquid into an inside and a housing member having a discharge channel configured to discharge the test liquid to an outside, and a cell capture filter disposed on a channel between the introduction channel and the discharge channel inside the chassis and including a filter region therein provided with a through-hole in a thickness direction and configured to pass the test liquid, the method for manufacturing a cell capture device comprising the step of: forming the lid member including a looped first protrusion part provided at a position outside of the filter region and inside of an outer edge of the cell capture filter in an assembled state and protruding outward from a surface of a side to which the cell capture filter is attached; forming the housing member including a looped second protrusion part provided at a position corresponding to the first protrusion part in the assembled state and located at a position corresponding to the first protrusion part to protrude; and fixing the lid member, the housing member, and the cell capture filter by sandwiching the cell capture filter between the first protrusion part and the second protrusion part.

In the cell capture device, the cell capture filter, and the method for manufacturing a cell capture device, the cell capture filter is fixed by the first protrusion part provided in the lid member and the second protrusion part provided in the housing member of the cell capture device at the position outside of the filter region. As described above, the cell capture filter is fixed between the lid member and the housing member without being folded in a state where the filter region of the cell capture filter is surrounded by the looped first protrusion part and the looped second protrusion part. This results in generating no wrinkles in the cell capture filter and enables cells contained in the test liquid passing through the filter region inside the cell capture filter to be suitably captured.

The cell capture device can also have an aspect in which the lid member further includes a first fitting part provided at a position outside of and away from the first protrusion part and not overlapped with the cell capture filter in the assembled state, and fitted with the housing member, the housing member further includes a second fitting part fitted with the first fitting part of the lid member in the assembled state, and one of the first fitting part and the second fitting part forms a projection shape and the other forms a recess shape.

The cell capture filter can also have an aspect in which the lid member further includes a first fitting part provided at a position outside of and away from the first protrusion part and not overlapped with the cell capture filter in the fixed state, and fitted with the housing member, the housing member further includes a second fitting part fitted with the first fitting part of the lid member in the fixed state, one of the first fitting part and the second fitting part forms a projection shape and the other forms a recess shape, and the outer edge of the cell capture filter is located inside of a smaller inner periphery of either the first fitting part or the second fitting part and outside of a larger outer periphery of either the first protrusion part or the second protrusion part.

The method for manufacturing the cell capture device can also have an aspect including the steps of: forming the lid member further including a first fitting part provided at a position outside of and away from the first protrusion part and not overlapped with the cell capture filter in the assembled state, and fitted with the housing member; forming the housing member further including a second fitting part fitted with the first fitting part of the lid member in the assembled state; forming a projection shape in one of the first fitting part and the second fitting part and forming a recess shape in the other; and fitting the lid member, the housing member, and the cell capture filter so that the first protrusion part and the second protrusion part are located at the corresponding position to sandwich the cell capture filter and the first fitting part and the second fitting part are fitted with each other to fix the cell capture filter outside of the outer edge of the cell capture filter.

As described above, the first fitting part of the lid member and the second fitting part of the housing member are fitted with each other outside of the cell capture filter, so that the lid member, and the housing member are fitted outside of the outer edge of the cell capture filter, and thus the mechanical strength as the cell capture device is improved and the fluctuation in the height of the filter surface can be reduced.

The cell capture device can also have an aspect in which the cell capture device further includes a sealing member including an opening part in an inside, sandwiched between at least one of the first protrusion part and the second protrusion part and the cell capture filter, and having elasticity, the sealing member is located at all corresponding regions where the first protrusion part and the second protrusion part sandwich the cell capture filter, and a size of the opening part is formed in accordance with a shape of the inner periphery of the first protruding part and the second protruding part.

By providing the sealing member having elasticity at all corresponding regions where the first protrusion part and the second protrusion part sandwich the cell capture filter, the tight-sealing property of a channel in which the filter region of the cell capture filter is provided between the introduction channel and the discharge channel is improved and thus the fluctuation in the height of the filter surface of the cell capture filter can be reduced and accuracy in the capture and the observation of cells can be improved.

The cell capture device can also have an aspect in which the cell capture filter has a plurality of alignment holes at a position outside of the filter region and inside of the outer edge of the cell capture filter, and at least one of the lid member and the housing member has hole parts provided at positions corresponding to the alignment holes. The cell capture filter can also have an aspect in which the cell capture filter has a plurality of alignment holes at a position outside of the filter region and inside of the outer edge of the cell capture filter.

As described above, when the cell capture filter is provided with the alignment holes, by providing the hole parts at the positions corresponding to the alignment holes in the cell capture filter in at least one of the lid member and the housing member included in the cell capture device, at least one of the lid member and the housing member and the cell capture filter can be aligned well, and thus the repeatability of the support balance of the filter can be further improved.

A cell capture apparatus according to the present invention includes: the cell capture device described above; means for supplying a test liquid configured to supply the test liquid to the introduction channel of the cell capture device; means for supplying a processing liquid configured to supply, to the introduction channel of the cell capture device, the processing liquid for processing cells captured on the cell capture filter by passing through the cell capture filter; and means for selecting configured to select a liquid to be supplied to the cell capture device from the test liquid and the processing liquid.

According to the cell capture apparatus, by having the configuration for selecting the liquid to be supplied to the cell capture device by the means for selecting and, based on this result, supplying the test liquid or the processing liquid to the cell capture device, the capture process of cells in the test liquid can be carried out more effectively than conventional apparatuses.

### Advantageous Effects of Invention

According to the present invention, a cell capture device, a cell capture filter, and a cell capture apparatus that can capture and observe cells easily and accurately and can also reduce the fluctuation in the height of the filter surface, and a method for manufacturing the cell capture device are provided.

### Brief Description of Drawings

FIG. 1 is a schematic perspective view illustrating a configuration of a cell capture device.
FIG. 2 is a schematic perspective view of a lid member seen from above.
FIG. 3 is a schematic perspective view of the lid member seen from below.
FIG. 4 is a view illustrating the inner structure of the lid member and an arrow view of the IV-IV cross-section in FIG. 2.
FIG. 5 is a schematic perspective view of a housing member seen from above.
FIG. 6 is a schematic perspective view of the housing member seen from below.
FIG. 7 is a view illustrating the inner structure of the housing member and an arrow view of the VII-VII cross-section in FIG. 5.
FIG. 8 is a perspective view and a plane view of a fastening member.
FIG. 9 is a view illustrating the structure of a filter.
FIG. 10 is a disassembled perspective view illustrating the configuration of the cell capture device.
FIG. 11 is a view illustrating a configuration and a method for using a jig for attaching a sealing member.
FIG. 12 is a view illustrating a configuration and a method for using a jig at the time of attaching the cell capture filter to the housing member.
FIG. 13 is a view illustrating a configuration and a method for using the jig at the time of attaching the cell capture filter to the housing member.
FIG. 14 is a view illustrating the positional relation between the cell capture filter, the sealing member, and the lid member.
FIG. 15 is a view enlarging the left side of an arrow view of the XV-XV cross-section in FIG. 1 schematically illustrating a cross-section after assembling the lid member, the cell capture filter, the sealing member, and the housing member.
FIG. 16 is a view enlarging the left side of an arrow view of the XVI-XVI cross-section in FIG. 1 schematically illustrating a cross-section after assembling the lid member, the cell capture filter, the sealing member, and the housing member.
FIG. 17 is a view illustrating a method for mounting the fastening members.
FIG. 18 is a block diagram illustrating a configuration of a cell capture apparatus.
FIG. 19 is a schematic perspective view of a lid member according to a modified example seen from below.
FIG. 20 is a view illustrating the inner structure of the lid member according to the modified example and corresponding to the arrow view of the IV-IV cross-section in FIG. 2.
FIG. 21 is a schematic perspective view of a housing member according to the modified example seen from above.
FIG. 22 is a view illustrating the inner structure of the housing member according to the modified example and corresponding to the arrow view of the VII-VII cross-section in FIG. 5.
FIG. 23 is a view schematically illustrating the cross-section after assembling the lid member, the cell capture filter, the sealing member, and the housing member according to the modified example and corresponding to FIG. 15.

### Description of Embodiments

Hereinafter, the best mode for carrying out the present invention will be described in detail with reference to the attached drawings. In the description of the drawings, the same sign is assigned to the same element and redundant descriptions are omitted.

### Cell Capture Device

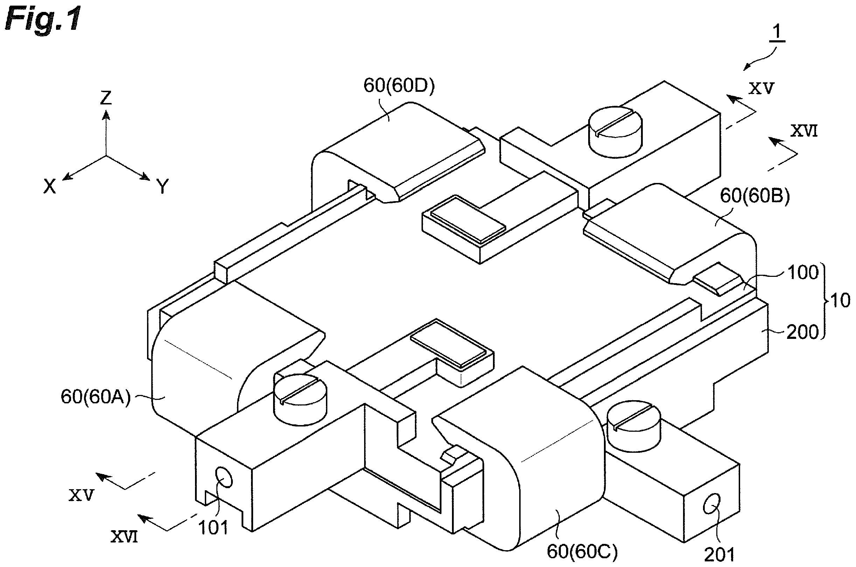

FIG. 1 is a schematic perspective view illustrating a configuration of a cell capture device according to an embodiment of the present invention. A cell capture device 1 illustrated in FIG. 1 has a function for capturing specific cells in a cell dispersion with a filter provided inside the cell capture device 1. As a particularly suitable example, the cell capture device 1 has a function in which red blood cells, white blood cells, and platelets (hereinafter, these are collectively called "blood cell components") are passed while circulating tumor cells called CTCs are captured from blood containing the CTCs. The cells captured with the cell capture filter are observed with a microscope or the like after applying staining processing on the cells by introducing a washing liquid, a staining liquid, and the like inside the device.

The cell capture device 1 illustrated in FIG. 1 is provided with a chassis 10 and a filter through which a test liquid passes and that is provided inside the chassis 10. The test liquid is a liquid in which cells are dispersed and specifically is blood and the like. The chassis 10 includes a lid member 100 and a housing member 200. In the chassis 10, the area of the region where through-holes of the cell capture filter are provided is determined to be 25 mm² to 900 mm². The area of the region where the through-holes are provided is more preferably 25 mm² to 400 mm² and further preferably 25 mm² to 100 mm².

The dimension and the shape of the part surrounded with a lid member side guide part or a housing member side guide part described below are preferably approximately square shape having a one side length of 10 mm to 100 mm in a plane view from the viewpoint of workability and observability. The one side length is more preferably 15 mm to 70 mm and further preferably 20 mm to 30 mm.

The lid member 100 and the housing member 200 are fixed to each other by clamping them from both sides in the overlapped direction (direction intersecting the cell capture filter) with four fastening members 60 (60A to 60D). The four fastening members 60 are located at the peripheral edge part of the chassis 10 so as to surround the cell capture filter.

As the material for the lid member 100 and the housing member 200, a material having relatively high rigidity is preferably selected. This allows deformation caused by applying pressure at the time of assembling and fastening the cell capture device 1 to be reduced. However, use of a material having excessively high rigidity may damage the filter at the time of assembling the cell capture device 1. The rigidity of the material can be represented by, for example, Young's modulus. Consequently, as the material of the lid member 100 and the housing member 200, a material having Young's modulus at room temperature of 0.1 GPa to 100 GPa is preferable and that of 1 GPa to 10 GPa is more preferable. Young's modulus can be measured by generally known Ewing's method.

The lid member 100 is preferably formed of a material having translucency to the light having a wavelength used at the time of detecting the cells that are targets of observation, particularly the light having a visible wavelength region. Examples of the material for the lid member 100 may include glass, quartz glass, plastics (particularly, acrylic resins), and macromolecules such as polydimethylsiloxane. The material, however, is not limited to these materials. The lid member 100 and the housing member 200 are not necessarily formed of the same material. When each of the lid member 100 and the housing member 200 has a fitting part, however, the materials are preferably the same from the viewpoint that the stress applied to the fitting part at the time of the assembly is equally applied to the lid member 100 side and the housing member 200 side. As the materials for the lid member 100 and the housing member 200, an acrylic resin having low autofluorescence property is preferable and polymethyl methacrylate is particularly preferable because this material enables the device to be produced in a large quantity. Generally, when cancer cells are observed, staining processing is carried out on the target cells with a fluorescence reagent and thereafter the processed cells are irradiated with the light having a wavelength of ultraviolet or visible light of 300 nm to 800 nm to carry out fluorescence observation. Therefore, as the lid member 100, a material having low autofluorescence is preferably selected so that the material itself does not emit light at the time of irradiation with the light in a range of ultraviolet or visible light (this phenomenon is called "low autofluorescence property"). Generally, organic macromolecules having aromatic rings such as a polystyrene resin and a polycarbonate resin have a high autofluorescence property and thus these macromolecules are not suitable for the above purpose in many cases.

The lid member 100 has introduction channels 101 and 102 for introducing the test liquid containing the cells that are targets of observation and the processing liquid for staining processing of the cells captured on the cell capture filter into the inside. The housing member 200 has a discharge channel 201 for discharging the test liquid from the inside to the outside. In the following description, a direction of attaching the introduction channels 101 and 102 is defined as an X axis direction, a horizontal direction orthogonal to the X axis is defined as a Y axis direction, and a vertical direction orthogonal to the X axis and Y axis is defined as a Z axis direction.

Subsequently, the configuration of the cell capture device 1 will be described in detail. First, each configuration of the lid member 100, the housing member 200, the fastening members 60, and the cell capture filter will be described and thereafter the cell capture device formed by assembling these components will be described.

### Lid Member

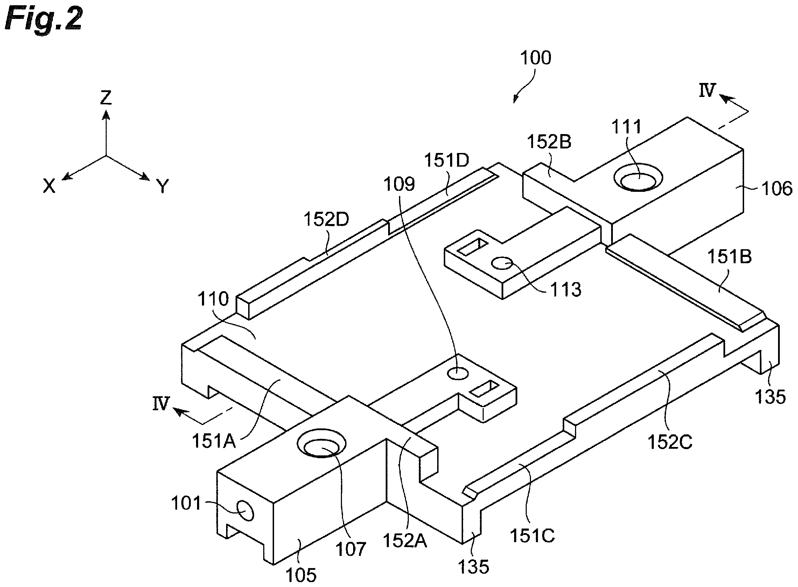

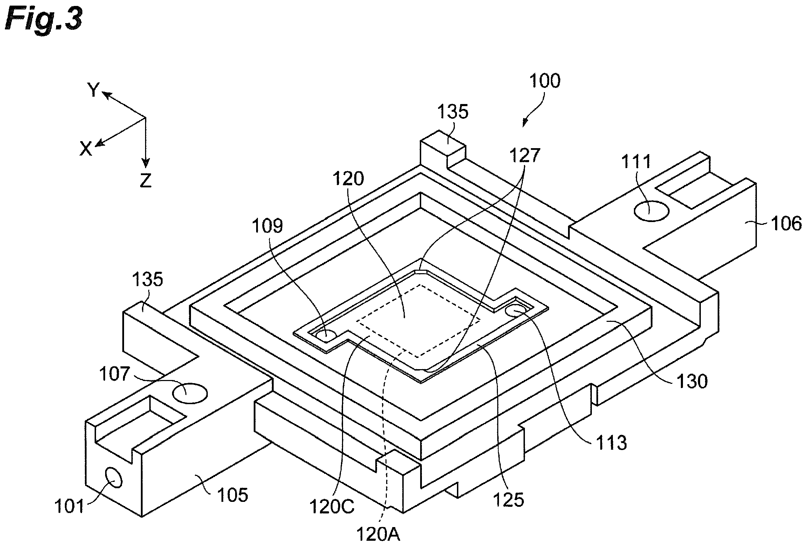

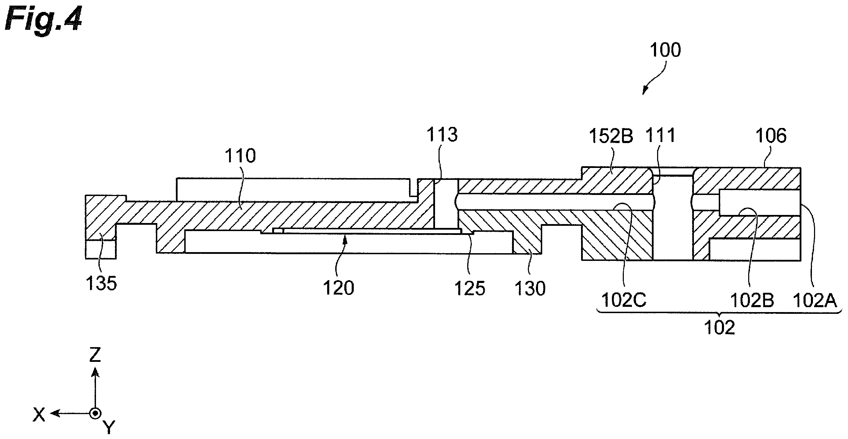

First, the lid member 100 will be described. FIGs. 2 to 4 are views illustrating a configuration of the lid member 100. FIG. 2 is a schematic perspective view of the lid member 100 seen from above. FIG. 3 is a schematic perspective view of the lid member 100 seen from below. FIG. 4 is an arrow view of the IV-IV cross-section in FIG. 2.

As illustrated in FIGs 2 to 4, in the lid member 100, two channel parts 105 and 106 are attached to a main body part 110 formed of a plate member having an approximately square shape so as to protrude along the X axis direction from the side edge of the main body part 110 to the outside. The channel parts 105 and 106 are each attached at a position apart from the center along the Y axis direction so that the channel parts 105 and 106 are located at positions different from each other when the lid member 100 is seen from the X axis direction. Here, the channel part 106 illustrated in FIG. 4 will be described. The channel part 105, however, has a configuration similar to that of the channel part 106.

In the lid member 100, an introduction region 120 formed of a recess type space in order that the test liquid passes through the cell capture filter is provided. The introduction region 120 is provided in the upper position of the filter region in the cell capture filter so that the region (filter region) providing through-holes in the cell capture filter attached between the lid member 100 and the housing member 200 is included when the cell capture device 1 is seen from above. To the corners of the introduction region 120, the introduction channels 101 and 102 are connected through perforations 109 and 113 for connection. The introduction region 120 is a space for guiding the test liquid or the processing liquid introduced from the introduction channels 101 and 102 to the through-holes of the cell capture filter.

Here, the introduction channel 102 will be further described using FIG. 4. The channel part 106 is provided with the introduction channel 102 extending along the X axis inside the channel part 106. The introduction channel 102 includes an introduction opening 102A, a channel 102B extending from the introduction opening 102A to the inside, and a channel 102C connecting to the channel 102B and having a smaller hole diameter than the hole diameter of the channel 102B. In addition, a perforation 111 passing through the channel part 106 in the vertical direction (Z axis direction) is provided between the channel 102B and the channel 102C (on the other hand, a perforation 107 is provided in the channel part 105). In the perforation 111, a valve provided with a perforation functioning as a channel opening and closing valve (channel opening and closing mechanism) is inserted. This allows the opening and closing of the channel 102B to be carried out by this valve. A perforation 113 for connecting the introduction region 120 and the introduction channel 102 is further provided at the edge part on the main body part 110 side of the introduction channel 102 so as to communicate with the introduction channel 102. This allows the liquid (the test liquid or the processing liquid) introduced from the introduction opening 102A to be transferred to the introduction region 120 through the channel 102B, the channel 102C, and the perforation 113.

At the peripheral edge of the introduction region 120, a first protrusion part 125 for preventing the liquid introduced into the introduction region 120 from leaking outside is provided so as to extend from the main body part 110 toward the lower position. As illustrated in FIG. 3, when the lid member 100 is seen from below, the first protrusion part 125 forms a looped shape protruding in the XY plane so that the looped shape includes the introduction region 120 and the region connected to the perforations 109 and 113 communicating with the introduction channels 101 and 102. It is important that this protrusion shape is continuously formed in a looped manner without discontinuous part. The height of the protruded shape preferably has the same height in any parts. When the height has fluctuation, a gap is generated between the first protrusion part 125 and the cell capture filter at the time of sandwiching the cell capture filter to cause leak.

In the lid member 100, a projection shaped first fitting part 130 formed of a projection part having a quadrilateral shape is formed so that the first fitting part 130 is provided away from the first protrusion part 125 and surrounds the peripheral edge of the first protrusion part 125. From the viewpoint of the strength of the lid member 100 and the fitting property of a cell capture filter 30, the length of the one side of the first fitting part 130 in the X axis direction or the Y axis direction is preferably 8 mm to 45 mm, more preferably 10 mm to 25 mm, and further preferably 15 mm to 30 mm. The width of the projection part constituting each side of the first fitting part 130 is preferably 1 mm to 5 mm, more preferably 1 mm to 3 mm, and further preferably 1.5 mm to 2 mm. As illustrated in FIG. 4, the first fitting part 130 is higher than the first protrusion part 125.

In addition, only on the side extending along the Y axis direction in the outer edge of the main body part 110, alignment walls 135 protruding toward the lower position (toward the upper position in FIG. 3) are provided. These alignment walls 135 are used for aligning and orienting the lid member 100 and the housing member 200 at the time of assembling the cell capture device 1.

The shape of the introduction region 120 and the first protrusion part 125 of the lid member 100 will be described in detail. In the introduction region 120 delimited by the first protrusion part 125, in two corners 127 different from two corners providing regions connected to the perforations 109 and 113 communicating with the introduction channels 101 and 102, the side surfaces constituting the inner wall surface of the introduction region 120 in the first protrusion part 125 of approximately quadrilateral shape have what is called chamfered shape. This structure is a structure for preventing accumulation of air bubbles in a hollow part inside the chassis 10 including the introduction region 120.

Referring back to FIG. 2, in the main body part 110 having approximately square shape, lid member side guide parts 151 (151A to 151 D) extending in an anti-clockwise direction in the plane view along the peripheral edge parts from respective ends (respective corners) as starting points are provided at the four-side peripheral edge parts of the surface to be an upper surface in an assembled state. The lid member side guide parts 151 are protrusions protruding toward the upper position of the lid member 100 and define a direction where the fastening members 60 slide by engaging the claws of the fastening members 60 described below with the guide parts. In the peripheral edge parts to which the channel parts 105 and 106 are attached at the side edge among the four peripheral edge parts, each of the lid member side guide parts 151 A and 151 B is provided to the side having longer distance in the peripheral edge parts divided by the channel parts 105 and 106. In other words, the lid member side guide part 151A and the lid member side guide part 151B are provided at positions diagonal to each other with respect to the center of the main body part 110 in the plane view. On the other hand, in the remaining peripheral edge parts to which the channel parts 105 and 106 are not attached, each of the lid member side guide parts 151C and 151D is disposed on the side to which a channel part being one of the channel parts 105 and 106 located at the front side is attached when the main body part 110 is seen from the Y axis direction. In other words, the lid member side guide part 151C and the lid member side guide part 151D are provided at positions diagonal to each other with respect to the center of the main body part 110 in the plane view. As illustrated in FIG. 2, the stepped parts of the starting points of the lid member side guide parts 151 and the upper surface of the main body part 110 are chamfered obliquely.

The lid member side guide parts 151 (151A to 151D) extend from the positions close to the edge parts of the main body part 110 as the starting points to approximately center parts in the respective peripheral edge parts of the main body part 110. From the approximately center parts, lid member side stoppers 152 (152A to 152D) are continuously provided and continuously extend along the peripheral edge parts. The lid member side stoppers 152A to 152D are protrusions higher than the lid member side guide parts 151A to 151D and define limit positions where the fastening members 60 described below slide along the lid member side guide parts 151A to 151D. The lid member side stoppers 152A to 152D extend to the positions close to the edge parts opposite to the starting points of the lid member side guide parts 151A to 151D in the respective peripheral edge parts. The parts of the lid member side stoppers 152A and 152B in the peripheral edge parts to which the channel parts 105 and 106 are attached are integrated with the base parts of the channel parts 105 and 106.

The widths of the lid member side guide parts 151A and 151B and the lid member side stoppers 152A and 152B in the X axis direction are determined to be larger than the widths of the lid member side guide parts 151C and 151D and the lid member side stoppers 152C and 152D in the Y axis direction.

### Housing Member

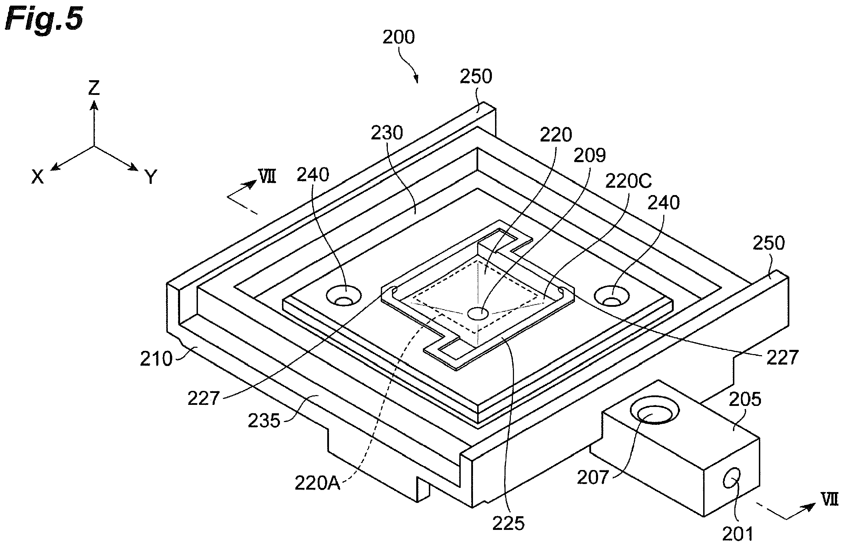

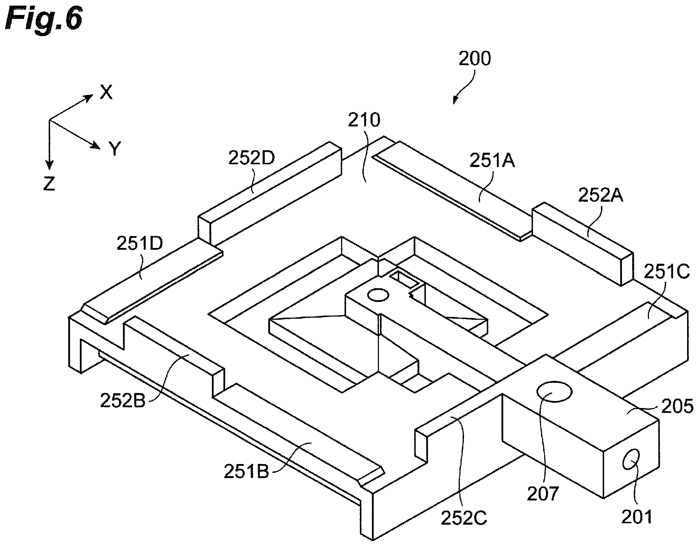

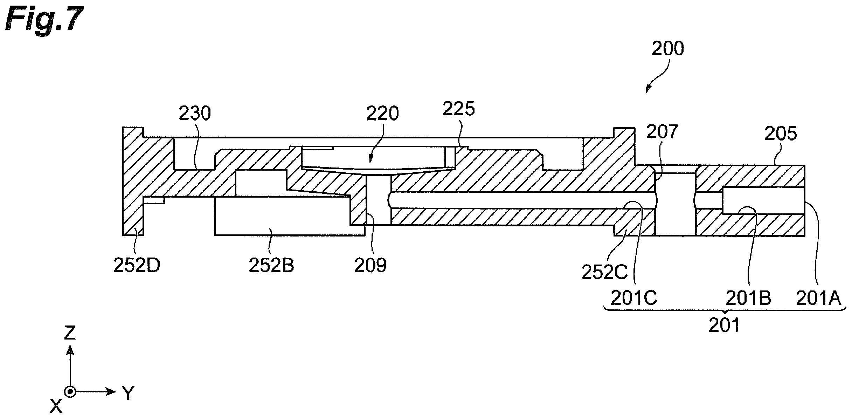

Subsequently, the housing member 200 will be described. FIGs. 5 to 7 are views illustrating a configuration of the housing member 200. FIG. 5 is a schematic perspective view of the housing member 200 seen from above. FIG. 6 is a schematic perspective view of the housing member 200 seen from below. FIG. 7 is an arrow view of the VII-VII cross-section in FIG. 5.

As illustrated in FIGs. 5 to 7, in the housing member 200, a channel part 205 protruding along the Y axis direction from the side edge of the main body part 210 to the outside is attached to a main body part 210 formed of a plate member having an approximately square shape. The channel part 205 is attached to the center of the side edge of the main body part 210.

The housing member 200 is provided with a discharge region 220 formed of a recess type space through which the test liquid passed through the cell capture filter passes. The discharge region 220 is provided at the lower position of the region to which the cell capture filter is attached so that the region (the filter region) where the through-holes are provided in the cell capture filter attached to the housing member 200 is included when the cell capture device 1 is seen from above. The discharge region 220 is connected to the discharge channel 201 through a perforation 209 for connection and is a space for discharging the test liquid through the discharge channel 201 to the outside.

The discharge channel 201 will be further described using FIG. 7. Inside the channel part 205, the discharge channel 201 extending along the Y axis is provided. The discharge channel 201 includes a discharge opening 201 A, a channel 201B extending from the discharge opening 201A to the inside, and a channel 201C connecting to the channel 201B and having a smaller hole diameter than the hole diameter of the channel 201B. In addition, a perforation 207 passing through the discharge channel 201 in the vertical direction (the Z axis direction) is disposed on the channel 201C. In the perforation 207, a valve provided with a perforation functioning as a channel opening and closing valve is inserted. This allows the opening and closing of the channel 201C to be carried out by this valve. In addition, a perforation 209 for connecting the discharge region 220 and the discharge region 201 is provided at the edge part on the main body part 210 side of the discharge region 201 so as to communicate with the discharge channel 201. This allows the liquid (the test liquid or the processing liquid) reached the discharge region 220 through the cell capture filter to be transferred to the discharge opening 201 A through the perforation 209, the channel 201C, and the channel 201B.

At the peripheral edge of the discharge region 220, a second protrusion part 225 for reducing the fluctuation in the height of the cell capture filter in the XY plane to be planarized is provided so as to extend from the main body part 210 toward the upper position. As illustrated in FIG. 5, when the housing member 200 is seen from above, the second protrusion part 225 forms an shape corresponding to the first protrusion part 125 of the lid member 100 in the XY plane.

A recess-shaped second fitting part 230 is formed of a recess part having a quadrilateral shape so that the second fitting part 230 is provided away from the second protrusion part 225 and surrounds the peripheral edge of the second protrusion part 225. The second fitting part 230 is provided so as to correspond to the first fitting part 130 of the lid member 100.

A region that is located between the second protrusion part 225 and the second fitting part 230 and in which a region 225A protruding outward of the discharge region 220 is not provided is provided with two bottomed holes (hole parts) 240 in positions diagonal to each other. The bottomed holes 240 are used for aligning the housing member 200 and the cell capture filter 30 as described below. The bottomed holes 240 may be through-holes passing through the housing member. In this embodiment, the example in which the bottomed holes 240 are disposed on the housing member 200 side is described. However, the bottomed holes 240 may be disposed on the lid member 100 side.

The difference of dimensions between the second fitting part 230 of the housing member 200 and the first fitting part 130 of the lid member 100 is preferably determined in consideration of the thickness of the cell capture filter 30. The difference between the inside of the second fitting part 230 and the inside of the first fitting part 130 (the difference of the inside of the fitting parts seen from the center of the member) is preferably 0.005 mm to 0.3 mm, more preferably 0.005 mm to 0.2 mm, and further preferably 0.005 mm to 0.1 mm. When the difference between the inside of the second fitting part 230 and the inside of the first fitting part 130 is thinner than the thickness of the cell capture filter, the gap is smaller than the thickness of the cell capture filter. However, the lid member and the housing member formed of plastics are relatively elastic and fixing force when the cell capture filter is fitted is increased and thus this configuration is preferable.

In the housing member 200, in addition to the second fitting part 230, alignment grooves 235 are provided only in the direction orthogonal to the direction extending to the outer edge providing the channel part 205 in the outer edges of the main body part 210 (the X axis direction), that is, the side extending along the Y axis direction. These alignment grooves 235 are used for aligning and orienting the lid member 100 and the housing member 200 at the time of assembling the cell capture device 1. In the same direction as the direction where the outer edge providing the channel part 205 in the outer edges of the main body part 210 extends (the X axis direction), covering parts 250 extending toward the upper position and covering the side parts of the lid member 100 are provided.

In the discharge region 220 delimited by the second protrusion part 225, in two corners 227 different from two corners providing regions connected to the perforations 109 and 113 communicating with the introduction channels 101 and 102 in the lid member 100 when the lid member 100 is combined, the side surfaces constituting the inner wall surface of the introduction region 120 in the first protrusion part 125 of approximately quadrilateral shape have what is called chamfered shape.

As illustrated in FIG. 6, in the main body part 210 having the approximately square shape, housing member side guide parts 251 (251A to 251D) extending along peripheral edge parts from respective ends (respective corners) as starting points are provided at the four-side peripheral edge parts of the surface to be a lower surface (a surface exposed to the outside) in the assembled state. The housing member side guide parts 251A to 251D are protrusions protruding toward the lower position (toward the upper position in FIG. 6) of the housing member 200 and define a direction where the fastening members 60 slide by engaging the claws of the fastening members 60 described below with the guide parts. The housing member side guide parts 251A to 251D are provided at positions corresponding to the lid member side guide parts 151A to 151D, respectively, in the thickness direction of the chassis 10 at the time of assembling the housing member 200 and the lid member 100. For example, in the peripheral edge part to which the channel part 205 is attached, the housing member side guide part 251C is provided at the side being the right side illustrated in FIG. 6 when the channel part 205 is seen from the Y axis direction as the front side with respect to the main body part 210 in FIG. 6. As illustrated in FIG. 6, stepped parts of the starting points of the housing member side guide parts 251A to 251D and the upper surface of the main body part 210 are chamfered obliquely.

The housing member side guide parts 251 (251A to 251D) are extended from the positions close to the edge parts of the main body part 210 as the starting points to approximately center parts in the respective peripheral edge parts of the main body part 210. From the approximately center parts, housing member side stoppers 252 (252A to 252D) are continuously provided and continuously extend along the peripheral edge parts. The housing member side stoppers 252A to 252D are protrusions higher than the housing member side guide parts 251A to 251D, define limit positions where the fastening members 60 described below slide along the housing member side guide parts 251A to 251D, and function as the legs of the cell capture device 1 at the time of use and observation of the cell capture device 1 (refer to FIG. 7). The parts of the housing member side stoppers 252A to 252D extend to the positions close to the edge parts opposite to the starting points of the housing member side guide parts 252A to 252D in the respective peripheral edge parts. The housing member side stopper 252C in the peripheral edge part to which the channel part 205 is attached is integrated with the base part of the channel part 205.

When the lid member 100 and the housing member 200 are formed of plastics such as an acrylic resin, these members can be manufactured by injection molding. The method for manufacturing the lid member 100 and the housing member 200, however, is not limited to the manufacturing method described above.

### Fastening Member

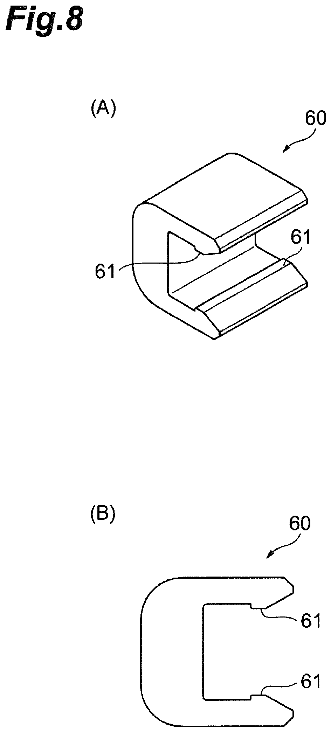

Subsequently, the fastening member 60 will be described. FIG. 8(A) is the perspective view of the fastening member. FIG. 8(B) is the front view of the fastening member 60. The fastening member 60 is an integrally molded member forming a shape having a transverse cross-section in which one face of the side part of the tubular body having an approximately quadrilateral shape is opened (that is, a U shape of the transverse cross-section) and fastens the lid member 100 and the housing member 200 by clamping so as to press from both sides in a direction where these members are overlapped (a direction intersecting with the cell capture filter).

At the inner surface of the U shape and the tip parts of the U shape, the fastening member 60 has claws 61 and 61 protruding facing each other in a direction where the lid member 100 and the housing member 200 should be clamped. These claws 61 and 61 extend to a direction corresponding to the height of the "tubular body having the approximately quadrilateral shape". In other words, the claws 61 and 61 extend to a direction along the peripheral edge parts of the lid member 100 and the housing member 200 when the fastening members 60 are located at the peripheral edge part of the chassis 10 and can slide along the lid member side guide parts 151 and the housing member side guide parts 251 by engaging with the lid member side guide parts 151 and the housing member side guide parts 251. From the viewpoint of more uniformly pressing, the length of the fastening member 60 in the sliding direction is preferably 3 mm to 20 mm, more preferably 4 mm to 15 mm, and further preferably 5 mm to 10 mm. With respect to the length of fastening member 60 in the sliding direction, the ratio of the length of fastening member 60 in the sliding direction to the length of the one side of the peripheral edge part of the chassis 10 equipped with the fastening member 60 is preferably 0.2 to 0.4.

From the viewpoint of uniformly pressing the peripheral edge part of the chassis 10 to fasten, identical fastening members are preferably used as the fastening members 60. For example, the fastening members 60 having the same shape and the same size or having less fluctuation of the applied pressure are preferably used.

As the material for the fastening member 60, a material having appropriate elasticity (2 GPa to 5 GPa as Young's modulus), excellent tensile strength (49 MPa or more), and elongation in a range of 10% to 100% is preferable and a polycarbonate resin or a polyacetal resin is preferable.

### Cell Capture Filter

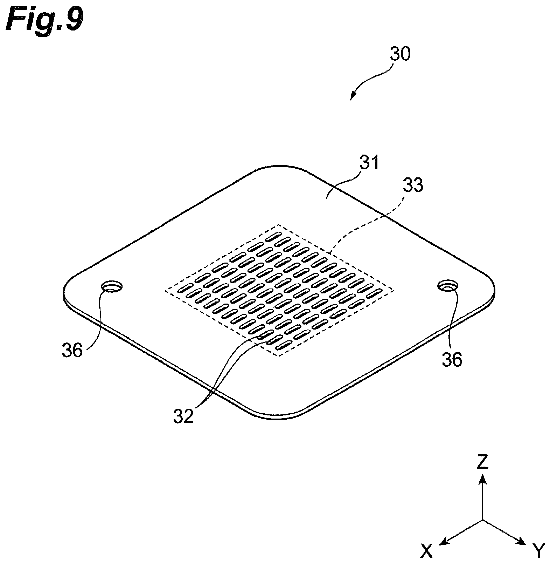

Subsequently, the cell capture filter 30 provided between the lid member 100 and the housing member 200 will be described using FIG. 9.

As illustrated in FIG. 9, the cell capture filter 30 includes a filter region 33 forming a plurality of through-holes 32 in a sheet 31 containing a metal as a main component in a thickness direction to pass the test liquid. The sheet 31 has an approximately square shape and the four corners are rounded. The shape of the through-holes 32 in the surface of the sheet 31 is an approximately rectangle, in which each short side is an arc shape, (hereinafter, referred to as an approximately rectangular shape). The through-holes 32 are formed such that the long sides are along the X axis direction. Outside of the filter region 33, the alignment holes 36 of the cell capture filter 30 are provided. The alignment holes 36 are used for aligning the housing member 200 with the cell capture filter 30 and are provided at positions corresponding to the bottomed holes 240 of the housing member 200.

The material of the sheet 31 is preferably a metal as a main component. The main component means a component having the highest ratio in the material forming the sheet 31. By using the metal as the main component of the material of the sheet of the cell capture filter 30, the fluctuation in the size of the through-holes is smaller and thus cells can be separated and concentrated in high accuracy. In addition, metal is more rigid than any other materials such as plastics and thus a size or a shape tends to be retained even when force is externally applied. Therefore, it is considered that separation and concentration in high accuracy are possible by deforming the blood components having slightly larger size than the hole diameter of the through-hole to pass through the through-holes. Here, the "concentration" means that an existence ratio of the number of the cells being the target for separation and concentration is increased before and after passing the test liquid through the cell capture device.

For example, when the test liquid is blood and CTCs are captured, the existence ratio of CTCs to the white blood cells and the like is included as the target of the separation and concentration. In white blood cells, some cells having almost the same size as that of the through-holes exist and thus not all white blood cells can pass through the through-holes. In this case, however, the white blood cells have relatively larger deformation ability by pressure and thus the white blood cells can pass through-holes having smaller opening than the white blood cells themselves by external force such as suction or pressurization. As a result, the existence ratio of CTCs to the white blood cells after passing through the through-holes is higher than that before passing through the through-holes. The phenomenon is called the "concentration".

Examples of the material of the metal used for the sheet 31 may include gold, silver, copper, aluminum, tungsten, nickel, chromium, and alloys of these metals. The material of the metal, however, is not limited to these metals. The metal may be used singly or may be used as the alloy with other metals or the oxide of the metal for adding functionality. From the viewpoint of price and easy availability, nickel, copper, gold, and a metal containing these metals as a main component are preferably used. In particular, a metal containing nickel as a main component is preferably used. When the sheet 31 is formed of the material containing nickel as the main component, the surface of the nickel is preferably plated with gold. The gold plating can prevent oxidation of the filter surface and thus adhesion of the cells and the blood cell components to cell capture filter becomes uniform. This allows the repeatability of data to be improved.

The thickness of the cell capture filter 30 is preferably 3 µm to 100 µm and more preferably 10 µm to 30 µm. When the film thickness is determined to be 3 µm to 100 µm, the filter is easily handled and is adequate for high-precision processing. The height difference of the plane of the filter region in the filter is preferably 16 µm or less, where the height difference is determined as the difference between the maximum and the minimum in focal distances at the time of observing the five places in total of the ends and the center parts of the filter surfaces with a microscope.

The size of the cell capture filter 30 depends on the size of the cell capture device 1. Specifically, the size is a size where the outer edge of the cell capture filter 30 is located outside of the first and second protrusion parts and the outer edge of the cell capture filter 30 is located inside of the alignment walls 135 and covering parts 250 of the cell capture device 1. The size of the cell capture filter 30 is smaller than the inner peripheries of first fitting part 130 of the lid member 100 and the second fitting part 230 of the housing member 200 of the cell capture device 1 when the cell capture device 1 has the first and second fitting parts. This configuration allows fluctuation in the height of the filter surface of the cell capture filter 30 at the time of sandwiching the cell capture filter 30 between the lid member 100 and the housing member 200 to be reduced and the stability to be improved.

As the size of the cell capture filter 30, specifically, the length of one side of the approximately square shape is preferably 10 mm to 50 mm, where the corners of the approximately square shape are preferably rounded. The length is more preferably 12 mm to 30 mm and further preferably 13 mm to 20 mm.

In the cell capture filter 30, the size of the filter region 33 provided with the through-holes 32 and passing through the test liquid is preferably 25 mm² to 900 mm², more preferably 25 mm² to 400 mm², and further preferably 25 mm² to 100 mm². The size of the filter region 33 being equal to or less than 900 mm² allows a dead space to be reduced, while the size of the filter region 33 being equal to or more than 25 mm² allows the process time related to the test liquid to be shortened.

The size of the filter region 33 corresponds to the center part 120 A in the introduction region 120 of the lid member 100 illustrated in FIG. 3 and the center part 220A in the discharge region 220 of the housing member 200 illustrated in FIG. 5. In other words, the filter region 33 in the cell capture filter 30 is attached to the position corresponding to the center parts 120A and 220A described above.

The shape of the through-holes 32 provided in the cell capture filter 30 can be adequately changed. In the case of the through-holes 32 of approximately rectangular shape as illustrated in FIG. 9, for example, the range of the length of the short side can be determined to be about 5.0 µm to 15.0 µm. On the other hand, the length of the long side can be adequately changed depending on the size of the filter and the range of the length is about 10 µm to 300 µm. The hole diameter of the through-holes 32 provided in the cell capture filter 30 can be changed depending on the size of the capture-target cells. The hole diameter of the through-holes means a diameter of the largest sphere that can pass through each of the through-holes 32. For example, the hole diameter of the through-holes 32 in FIG. 9 can be the shorter side of the through-holes 32.

The shape of the through-holes 32 can be adequately changed. Examples of the shape may include wave-form-like through-holes formed by connecting edge parts of two perforations having a rectangular shape or a rounded rectangular shape (a rectangular shape having rounded-edge corners) in a predetermined angle. Representative examples of the shape of the through-holes may include a circular shape, a rectangular shape, a round rectangular shape, and a round-wave-like shape (a stlooped hole formed by connecting a plurality of semicircles at the edges alternately facing each other).

Examples of a method for manufacturing the filter in which the through-holes 32 having a small hole diameter in the cell capture filter 30 can be precisely formed may include a method for using metal plating using a photoresist. Specifically, the method for manufacturing the filter includes a step of laminating a photoresist on a metal foil; a step of overlapping a photomask having a translucent part similar to a shape of the through-hole on the photoresist to expose; a step of forming a photoresist pattern by developing the exposed photoresist and removing an uncured part of the photoresist; a step of plating the removed part of the photoresist pattern with a metal to form a metal plating pattern having lower height than the height of the photoresist pattern; a step of removing the metal foil by chemical dissolution to give a structure formed of the metal plating pattern and the photoresist pattern; and a step of removing the photoresist pattern from the structure to give a metal plating pattern (filter) having the through-holes 32 corresponding to the translucent part. The method for manufacturing the cell capture filter 30, however, is not limited to the above method.

### Method for Assembling Cell Capture Device

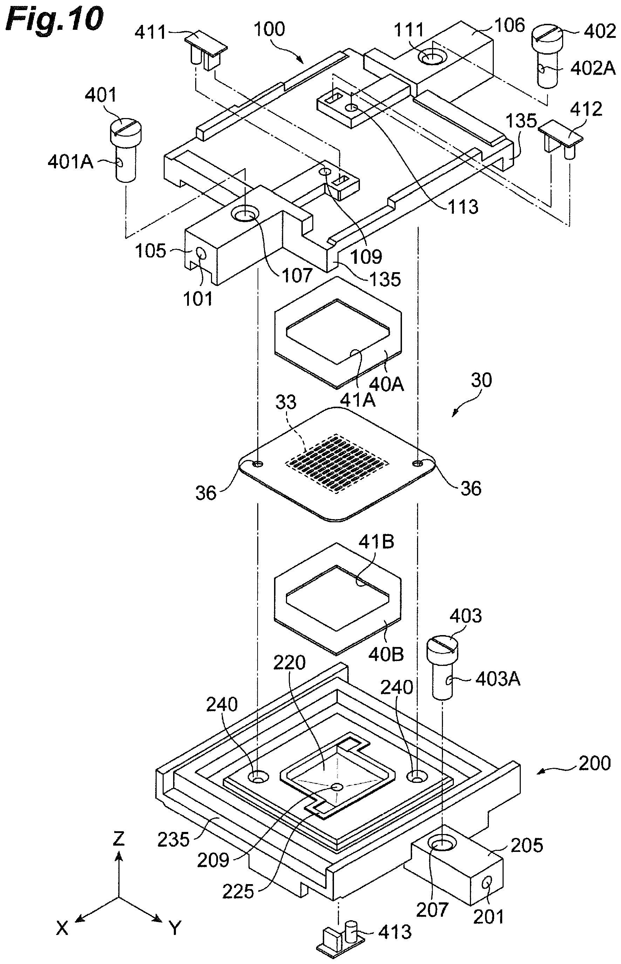

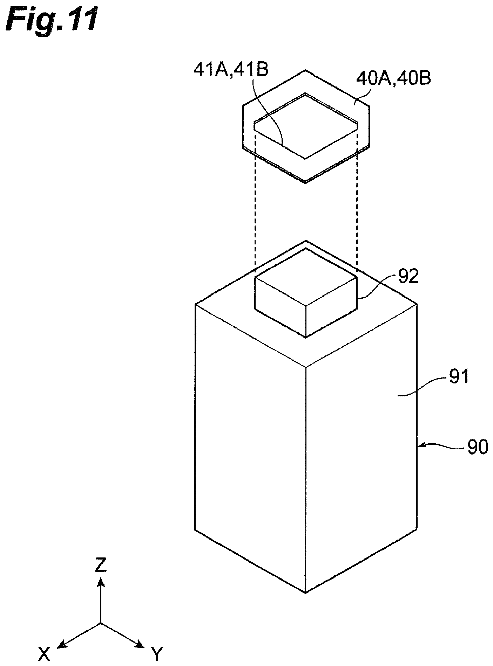

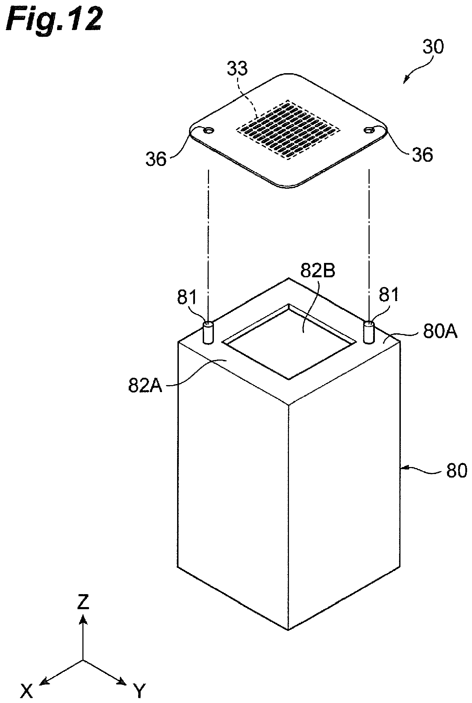

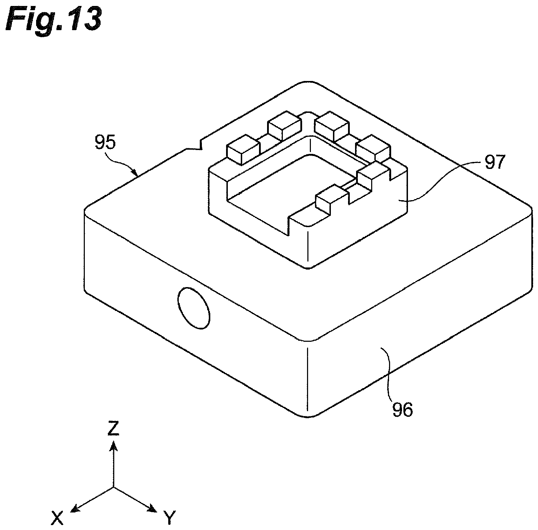

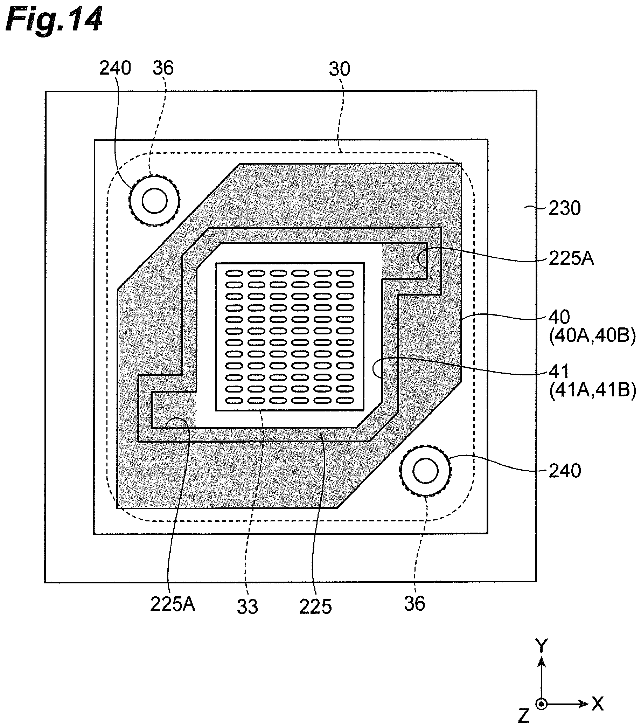

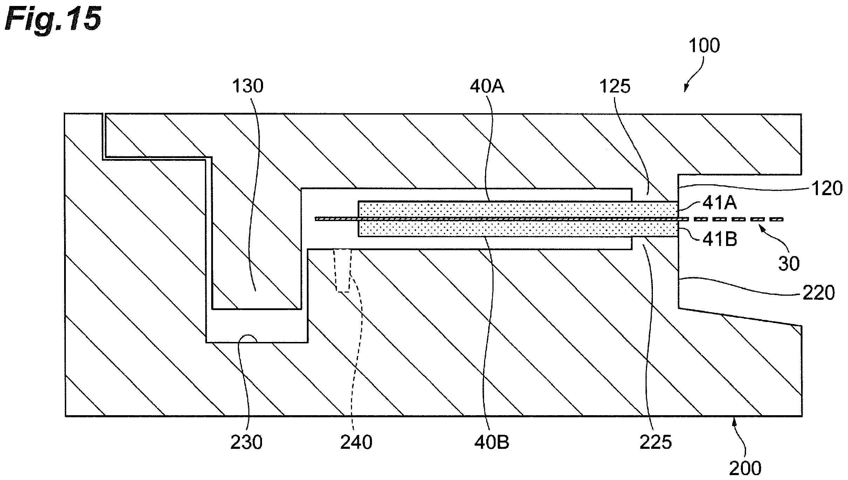

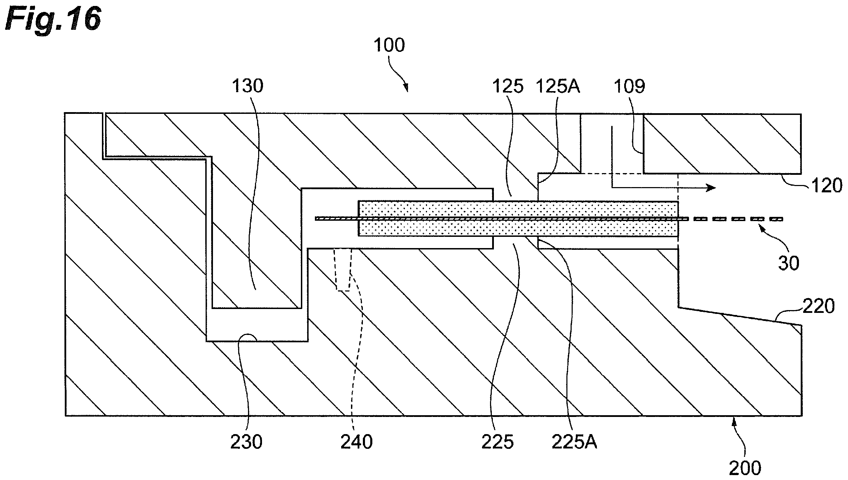

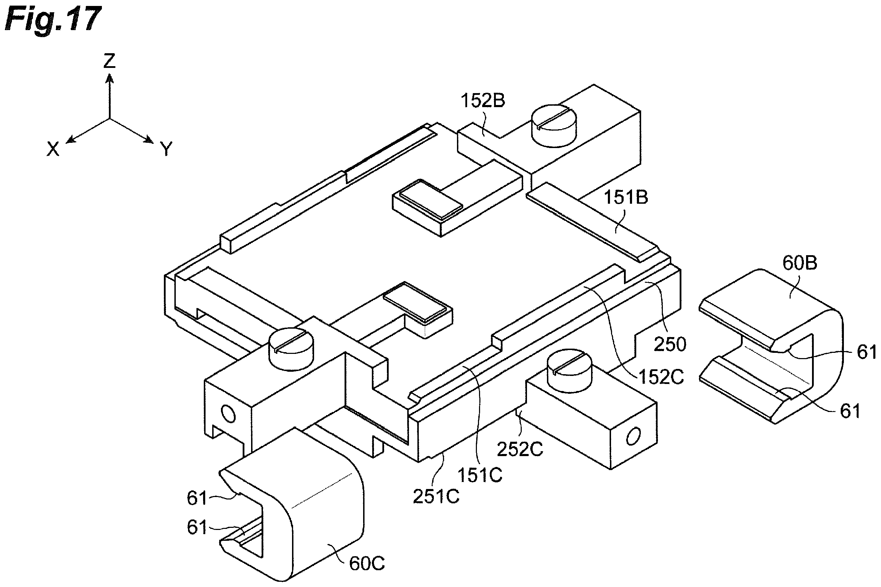

Subsequently, the method for assembling cell capture device 1 will be described using FIG. 10 to FIG. 17. FIG. 10 is a disassembled perspective view illustrating the configuration of the cell capture device 1. FIG. 11 is a view illustrating a configuration and a method for using a jig for attaching a sealing member 40 (a gasket). FIG. 12 is a view illustrating a configuration and a method for using a jig at the time of attaching the cell capture filter 30 to the housing member 200. FIG. 13 is a view illustrating a configuration and a method for using a jig at the time of attaching the cell capture filter 30 to the housing member 200. FIG. 14 is a view illustrating a positional relation between the cell capture filter 30, the sealing member 40 (the gasket), and the lid member 100 (housing member). FIG. 15 is a view enlarging the left side of an arrow view of the XV-XV cross-section in FIG. 1 schematically illustrating a cross-section after assembling the lid member 100, the cell capture filter 30, the sealing member 40 (the gasket), and the housing member 200. FIG. 16 is a view enlarging the left side of an arrow view of the XVI-XVI cross-section in FIG. 1 schematically illustrating a cross-section after assembling the lid member 100, the cell capture filter 30, the sealing member 40, and the housing member 200. FIG. 17 is a view illustrating a method for mounting the fastening members.

As an outline of the method for assembling the cell capture device 1, as illustrated in FIG. 10, the housing member 200, the sealing member 40B, the cell capture filter 30, the sealing member 40A, and the lid member 100 are stacked in this order and thereafter, as illustrated in FIG. 15 and FIG. 16, the housing member 200 and the lid member 100 are fitted. By mounting the fastening members 60 to the chassis 10 formed by fitting the lid member 100 and the housing member 200 as illustrated in FIG. 17, the lid member 100 and the housing member 200 are fixed to each other and the assembly of the cell capture device 1 is completed.

First, as illustrated in FIG. 10, the cell capture device 1 is assembled by stacking the lid member 100, the lid member side sealing member 40A, the cell capture filter 30, the housing member side sealing member 40B, and the housing member 200 in this order from upward to downward along the Z axis. At this time, the channel parts 105 and 106 of the lid member 100 are determined to extend along the X axis direction and the channel part 205 of the housing member 200 is determined to extend along the Y axis direction by setting the lid member 100 and the housing member 200 in a direction where the pair of the alignment walls 135 and the alignment grooves 235 are faced. This forms a configuration in which the channel parts 105, 106, and 205 protrude in different directions with each other.

The sealing members 40 (40A and 40B) are members sandwiched between the first protrusion part 125 of the lid member 100 and the filter 30 and between the second protrusion part 225 of the housing member 200 and the cell capture filter 30. The sealing members 40 are located whole regions where the first protrusion part 125 and the second protrusion part 225 exist and have a looped shape formed by punching out the center as the shape of the filter region 33 in order not to cover the filter region 33. The material of the sealing members 40 is not particularly limited as long as the material has elasticity and can maintain liquid-tight properties of the chassis 10. For example, silicone rubber is used. The thickness of the sealing members 40 is preferably 0.05 µm to 0.3 µm.

A method for attaching the sealing members 40A and 40B and the cell capture filter 30 between the lid member 100 and the housing member 200 is carried out as follows. First, the sealing member 40A is attached to the lid member 100 and the sealing member 40B is attached to the housing member 200 using a jig for attaching a sealing member 90 illustrated in FIG. 11. As illustrated in FIG. 11, the jig for attaching a sealing member 90 includes a main body part 91 extending in a columnar shape and a protrusion part 92 formed at one end of the main body part 91. The protrusion part 92 has the cross-section shape perpendicular to the extending direction of the main body part 91 corresponding to the opening 41A of the sealing member 40A and can fit to the introduction region 120 of the lid member 100. By inserting the protrusion part 92 of the jig for attaching a sealing member 90 into the opening 41A of the sealing member 40A, the sealing member 40A is located so as to surround the protrusion part 92. Thereafter, the jig for attaching a sealing member 90 is located at the position opposed to the upper side of the first protrusion part 125 of the lid member 100 to retain the jig for attaching a sealing member 90 in a state where the protrusion part 92 is fitted to the introduction region 120. Then, the sealing member 40A is transferred from the jig for attaching a sealing member 90 to the lid member 100. By this procedure, the sealing member 40A is easily attached to the upper part of the first protrusion part 125. The sealing member 40A transferred onto the lid member 100 adheres to the lid member 100 by adhesive force or electrostatic force and thus the possibility to cause displacement of the sealing member 40A from the transferred position is reduced.

By a method similar to the above method for attaching the sealing member 40A to the lid member 100, the sealing member 40B can be attached to the housing member 200. By these procedures, the two sealing members 40A and 40B are attached to the lid member 100 and the housing member 200, respectively.

Subsequently, the cell capture filter 30 is located at the position being the upper position of the discharge region 220 and the second protrusion part 225 of the housing member 200. FIG. 12 illustrates a jig for attaching a filter 80 used at the time of locating the cell capture filter 30. The jig for attaching a filter 80 is formed of a quadrilateral-shaped columnar member having an edge surface corresponding to the cell capture filter 30. In the jig for attaching a filter 80, pins 81 and 81 protruding from the edge surface are provided at the position corresponding to the alignment holes 36 and 36 of the cell capture filter 30. In the jig for attaching a filter 80, the edge surface 80A includes a recess-shaped center part 82B surrounded by an upper surface 82A recessed to the upper surface 82A where the pin 81 are formed. Either the upper surface 82A or the center part 82B has magnetic force and thus the jig for attaching a filter 80 is able to hold the cell capture filter 30 formed of a metal by the magnetic force. The reason why the center part 82B is recessed is to prevent the contact of filter region 33 to the upper surface 82A when the cell capture filter 30 contacts to the surface 82A by the magnetic force of the jig for attaching a filter 80. Consequently, the center part 82B is determined to be larger than the filter region 33.

FIG. 13 illustrates a jig for fixing a filter 95 used at the time of attaching the cell capture filter 30 to the housing member 200. The jig for fixing a filter 95 is a member attached to the housing member 200 from the lower surface side of the housing member 200 before the cell capture filter 30 is attached to the housing member 200. The jig for fixing a filter 95 includes a main body part 96 and a protrusion part 97 disposed on the main body part 96 and formed corresponding to the shape of the lower surface side of the housing member 200. The protrusion part 97 is determined to be the shape corresponding to the recess and the projection (refer to FIG. 6) formed at the position corresponding to the discharge region 220 and the periphery of the discharge region 220 on the lower surface side of the housing member 200. So as to generate magnetic force at the surface of the projection part corresponding to the recess part of the lower surface of the housing member 200 in the protrusion part 97, at least a part of the protrusion part 97 is formed to include a magnet. The magnetic force generated at the jig for fixing a filter 95 is determined to be stronger than the magnetic force generated at the jig for attaching a filter 80.

When the cell capture filter 30 is attached to the housing member 200, first, the cell capture filter 30 is attached to the jig for attaching a filter 80 and pins 81 are inserted into respective two alignment holes 36. At this time, the cell capture filter 30 is temporarily fixed to the jig for attaching a filter 80 by the magnet force of the upper surface 82A of the jig for attaching a filter 80. Subsequently, the edge surface 80A of the jig for attaching a filter 80 to which the cell capture filter 30 is attached is opposed to the housing member 200 to insert the pins 81 into two bottomed holes 240 of the housing member 200. By this procedure, the cell capture filter 30 is transferred to the housing member 200 side by the stronger magnetic force provided to the jig for fixing a filter 95 located at the lower surface of the housing member 200 and, as a result, the cell capture filter 30 is easily aligned. Thereafter, the housing member 200 in which the cell capture filter 30 and the sealing member 40B are aligned and the lid member 100 in which the sealing member 40A is aligned are faced each other, whereby the cell capture device 1 can be easily assembled.

The positions corresponding to the bottomed holes 240 and the alignment holes 36 in the housing member side sealing member 40B is cut out so that the cell capture filter 30 can be aligned using the jig for attaching a filter 80 (refer to FIG. 14). By carrying out the alignment as described above, the cell capture filter 30 can be located in high accuracy as well as the through-holes of the cell capture filter 30 can be easily and always located in the same predetermined direction at the time of assembling the cell capture device 1. This results in further improving the repeatability of cell capture.

FIG. 14 is a plan view inside of the second fitting part 230 at the time of attaching the sealing members 40A and 40B and the cell capture filter 30 to the housing member 200. As illustrated in FIG. 14, the openings 41A and 41B of the sealing members 40A and 40B are larger than the filter region 33 and the surface formed corresponding to the shape of the second protrusion part 225 (the upper surface of the region 225A protruding outward, however, is covered with the sealing members 40A and 40B. The outer periphery of the cell capture filter 30 and the outer periphery of the sealing members 40A and 40B are determined to be smaller than the second fitting part 230. The shape is determined that the alignment holes 36 and 36 of the cell capture filter 30 and the bottomed holes 240 and 240 of the housing member 200 are overlapped, but this region is not overlapped with the sealing members 40A and 40B.

As described above, to the housing member 200 attaching the sealing members 40A and 40B and the cell capture filter 30, the lid member 100 is located at the position where the first protrusion part 125 and second protrusion part 225 correspond and at the position where the first fitting part 130 and the second fitting part 230 correspond, and then, these members are approached in the vertical direction.

As illustrated in FIGs. 15 and 16, the lid member 100 and the housing member 200 can be approached to the position where the first protrusion part 125 and the second protrusion part 225 sandwich the cell capture filter 30 through the lid member side sealing member 40A and the housing member side sealing member 40B, respectively. At this time, the first fitting part 130 and the second fitting part 230 stop in a state where a part of the first fitting part 130 and a part of the second fitting part 230 are fitted. At this time, as illustrated in FIG. 15, the openings 41A and 41B of the sealing members 40A and 40B have a shape along the quadrilateral shape being an inner periphery of the first protrusion part 125 and the second protrusion part 225 (except the regions 125A and 225A protruding outward) and thus the inner wall of the introduction region 120 on the lid member 100 side, the opening 41 A of the sealing member 40A, the opening 41 B of the sealing member 40B, and the inner wall of the discharge region 220 on the housing member 200 side are formed so as to be flush in this order. In FIG. 15 and FIG. 16, the bottomed hole 240 is illustrated by a dashed line for reference.

As illustrated in FIG. 16, with respect to the regions 125A and 225A protruding outward in the first protruding part 125 and the second protruding part 225, the openings 41 A and 41B of the sealing members 40A and 40B have the shape not along the inner periphery shape of the first protrusion part 125 and the second protrusion part 225 and thus the sealing member 40A and sealing member 40B are exposed to the inner channel at the lower side of the perforation 109 forming the introduction channel. The test liquid or the processing liquid introduced from the perforation 109 is introduced to the introduction region 120 while being in contact with the upper sealing member 40A.

As illustrated in FIG. 15 and FIG. 16, the tight-sealing property of the inner introduction region 120 and the discharge region 220 is improved by sandwiching the cell capture filter 30 between the first protrusion part 125 and the second protrusion part 225 through the lid member side sealing member 40A and the housing member side sealing member 40B, respectively, regardless of whether the sealing members 40A and 40B are exposed to the inner space. Consequently, the test liquid or the processing liquid does not leak from the upper side and the lower side of the cell capture filter 30 to the outside.

The sizes of the cell capture filter 30 and the sealing members 40A and 40B located at the upper side and the lower side of the cell capture filter 30 are determined to eliminate overlap with the region where the first fitting part 130 of the lid member 100 and the second fitting part 230 of the housing member 200 are fitted and thus generation of the winkles or slacks of the cell capture filter 30 can be prevented by the effect of force at the time of fitting the lid member 100 and the housing member 200.

After the housing member 200 and the cell capture filter 30 are aligned using the jig for attaching a filter 80 and the jig for fixing a filter 95, the cell capture filter 30 is aligned to the housing member 200 but the position of the filter 30 is not fixed. Therefore, the cell capture filter 30 can respond to position change without physical barrier against the tensile force and the like until the cell capture filter 30 is finally fixed in the inside of the chassis 10, and thus the winkle and the like are more difficult to generate in a fixed state the filter 30 between the lid member 100 and the housing member 200.

The jigs illustrated in FIG. 11 to FIG. 13 are examples of jigs for attaching the sealing members 40A and 40B to the lid member 100 and the housing member 200 and for aligning using the alignment holes 36 and 36 of the cell capture filter 30 and the bottomed holes 240 and 240 of the housing member 200. Consequently, the jigs used for alignment and the like are not limited to the configurations illustrated in FIG. 11 to FIG. 13 and various modifications can be applied.

As described above, valves 401, 402, and 403 functioning as channel opening and closing valves are inserted into perforations 107, 111, and 207, respectively, after assembling the lid member 100 and the housing member 200 sandwiching the cell capture filter 30. These valves 401, 402, and 403 are provided with channel holes 401A, 402A, and 403A extending in a horizontal direction, respectively. When the valves are inserted into the perforations 107, 111, and 207, the channel holes of the valves correspond to the introduction channels 101 and 102 and the discharge channel 201, respectively. By rotating the valves, opening and closing of the channel holes and introduction channels (or discharge channel) can be switched. At the time of use, the leakage of the liquid is prevented and the introduction and discharge of the liquid are guided to appropriate directions by inserting stoppers 411, 412, and 413 into the perforations 109, 113, and 209, respectively.

Subsequently, a method for mounting the fastening members 60 will be described. As illustrated in FIG. 17, the fastening member 60C is approached to the lid member side guide part 151C and the housing member side guide part 251C corresponding to the lid member side guide part 151C from the X axis direction in a state where the U shape opening part faces the chassis 10 side to engage each of the claws 61 and 61 to the lid member side guide part 151C and the housing member side guide part 251C. Mounting the fastening member 60C is completed by sliding the fastening member 60C in the X axis direction. At this time, the fastening member 60C may slide until the side part of the fastening member 60C hits the side part of the lid member side stopper 152C and the side part of the housing member side stopper 252C or may stop sliding on the way without hitting the side parts of the stoppers.

Similarly, in order to mount the fastening member 60B to the chassis 10, the fastening member 60B is approached to the lid member side guide part 151B and the housing member side guide part 251 B corresponding to the lid member side guide part 151 B from the Y axis direction in a state where the U shape opening part faces the chassis 10 side to engage each of the claws 61 and 61 to the lid member side guide part 151B and the housing member side guide part 251B. Mounting the fastening member 60B is completed by sliding the fastening member 60B in the Y axis direction.

When the distances from the outer edge of the chassis 10 to the lid member side guide parts 151C and 151B are noticed, the distance from the outer edge of the chassis 10 to the lid member side guide part 151C is larger than the distance from the outer edge of the chassis 10 to the lid member side guide part 151B by the thickness of the covering part 250 of the housing member 200. By setting the widths of the lid member side guide part 151 B and the lid member side stopper 152B in the X axis direction larger than the widths of the lid member side guide part 151C and the lid member side stopper 152C in the Y axis direction as described above, the distance of the outer edge of the chassis 10 and the distance of parts of the lid member side guide parts 151 B and 151C where the claws 61 of the fastening members 60 are engaged are equal. In other words, each peripheral edge part of the chassis 10 can be equipped with the fastening members 60 having the same shape and the same size. Being able to use the fastening members 60 having the same shape and the same size as described above means being able to use identical fastening members 60 having uniform pressing force. This is preferable from the viewpoint of fastening the chassis 10 by pressing the peripheral edge part of the chassis 10 by uniform load.

Although not illustrated in FIG. 17, the chassis 10 can be equipped with the fastening members 60A and 60D in a similar way as described above. By equipping the chassis 10 with the four fastening members 60A to 60D as described above, the fastening members 60 can be located at the peripheral edge part of the chassis 10 so as to surround the cell capture filter 30.

With respect to the cell capture device 1 according to the above embodiment, the configuration of fastening the lid member 100 and the housing member 200 with the fastening members 60 is described as the example. The embodiment, however, is not limited to this configuration and a configuration of joining the lid member and the housing member be welding may be employed.

### Cell Capture Apparatus

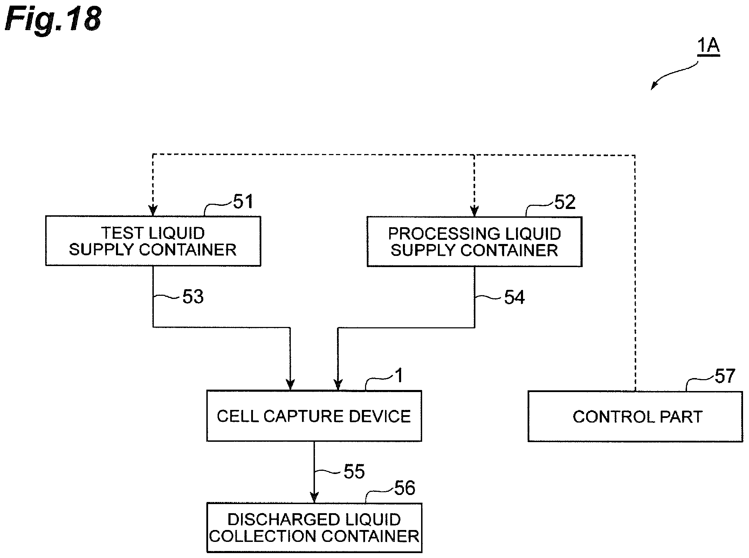

Subsequently, the cell capture apparatus using the cell capture device 1 will be described. FIG. 18 is a block diagram illustrating a configuration of the cell capture apparatus. As illustrated in FIG. 18, the cell capture apparatus 1A includes the cell capture device 1, a test liquid supply container 51 (means for supplying a test liquid) and a processing liquid supply container 52 (means for supplying a processing liquid) reserving the test liquid and the processing liquid that are supplied through each of the two introduction channels included in the cell capture device 1, a test liquid supply channel 53 (means for supplying a test liquid) connecting the test liquid supply container 51 and the cell capture device 1, a processing liquid supply channel 54 (means for supplying a processing liquid) connecting the processing liquid supply container 52 and the cell capture device 1, a discharge channel 55 connected to the discharge channel included in the cell capture device 1 and flowing the discharged liquid discharged from the discharge channel to the outside, a discharged liquid collection container 56 connected to the a discharge channel 55 and collecting the discharged liquid, and a control part 57 (means for selecting) selecting the liquid to be supplied to the cell capture device 1 from the test liquid and the processing liquid and controlling the channel switching and the like.

The control of the channel switching and the like are carried out by the control part 57. Specific examples of the method for switching the channel may include a method for opening and closing the channel opening and closing valve of the cell capture device 1. Liquid transfer in the cell capture apparatus 1A is also carried out by, for example, installing a peristaltic pump or the like to the channel. The example in which the one processing liquid is used is described. However, a configuration in which a plurality of processing liquid supply containers 52 are provided and the control part 57 controls which processing liquid is supplied to the cell capture device 1 may also be employed.

In the cell capture apparatus 1A, after completion of the cell capture process, the cells captured on the cell capture filter can be directly observed with a microscope from above the cell capture filter 1 by detaching the cell capture device 1 from the cell capture apparatus 1A in a state where the liquid flow inside the cell capture device is stopped by closing the channel opening and closing valves after carrying out the processing process for staining the captured cells.

As described above, the cell capture apparatus 1A can effectively carried out the capture process of the cells in the test liquid compared with the conventional apparatus by having the configuration in which the liquid to be supplied to the cell capture device 1 is selected by the control part 57 and, based on this result, the test liquid or the processing liquid is supplied to the cell capture device 1. In the cell capture apparatus 1 A using the cell capture device, after the capture process of the cells, presence or absence of the cells can be observed with improved workability by directly putting the cell capture device on the stage of the microscope without disassembling the cell capture device.

As described above, in the cell capture device and the method for manufacturing the cell capture device according to the embodiment, the cell capture filter 30 is fixed at the position outside of the filter region 33 by the first protrusion part 125 provided in the lid member 100 and the second protrusion part 225 provided in the housing member 200 in the cell capture device 1. The first fitting part 130 of the lid member 100 and the second fitting part 230 of the housing member 200 are fitted outside of the outer edge of the cell capture filter 30. As described above, the filter region 33 of the cell capture filter 30 is fixed between the lid member 100 and the housing member 200 in a state where the filter region 33 of the cell capture filter 30 is surrounded by the looped first protrusion part 125 and the looped second protrusion part 225 without folding the cell capture filter 30. Consequently, winkles and the like are not generated in the cell capture filter 30 and the cells contained in the test liquid passing through the filter region 33 in the cell capture filter 30 can be suitably captured. The lid member 100 and the housing member 200 are fitted outside of the outer edge of the cell capture filter 30, and thus the stability as the cell capture device 1 can be improved and the fluctuation in the height of the filter surface can be reduced.

The cell capture device 1 further includes the elastic sealing members 40 (40A and 40B) providing the openings 41 (41A and 41B) in the sealing members and sandwiched between the first protrusion part 125 and the second protrusion part 225, and the cell capture filter 30. The sealing members 40 are located at all regions corresponding to the regions formed by being sandwiched between the first protrusion part 125 and the second protrusion part 225, and the cell capture filter 30. The sizes of the openings 41 (41A and 41B) are formed in accordance with the shapes of the inner periphery of the first protrusion part 125 and the second protrusion part 225. This aspect allows the tight-sealing property (shielding property) of the channel where the filter region 33 of the cell capture filter 30 is provided between the introduction channel and the discharged channel to be improved. Consequently, the fluctuation in the height of the filter surface of the cell capture filter 30 can be reduced and the accuracy in the capture and the observation of cells can be improved. In order to improve the tight-sealing property (shielding property) of the channel where the filter region 33 of the cell capture filter 30 is provided, it is preferable that both sealing members 40A and 40B be provided. If at least one sealing member is provided, however, the tight-sealing property of the channel can be improved.

The cell capture device 1 can have an aspect in which the cell capture filter 30 has a plurality of alignment holes 36 and 36 at a position outside of the filter region 33 and inside of an outer edge of the cell capture filter 30, and at least one of the lid member 100 and the housing member 200 has hole parts (bottomed holes) 240 and 240 provided at positions corresponding to the alignment holes 36 and 36. As described above, when the cell capture filter 30 is provided with the alignment holes 36 and 36, at least one of the lid member 100 and the housing member 200 and the cell capture filter 30 can be aligned well by providing the hole parts at the positions corresponding to the alignment holes 36 and 36 in the cell capture filter 30 in at least one of the lid member 100 and the housing member 200 configuring the cell capture device 1 and thus the repeatability of the support balance of the filter can be further improved.

As described above, the embodiments of the present invention are described. The present invention, however, is not limited to the above embodiments and various modifications can be carried out. For example, although the shapes of the lid member and the housing member of the cell capture device are determined to be the approximately square shapes, the shapes may be a pentagon shape, a hexagon shape, or other polygonal shapes, or may be a circle shape or an ellipse shape.

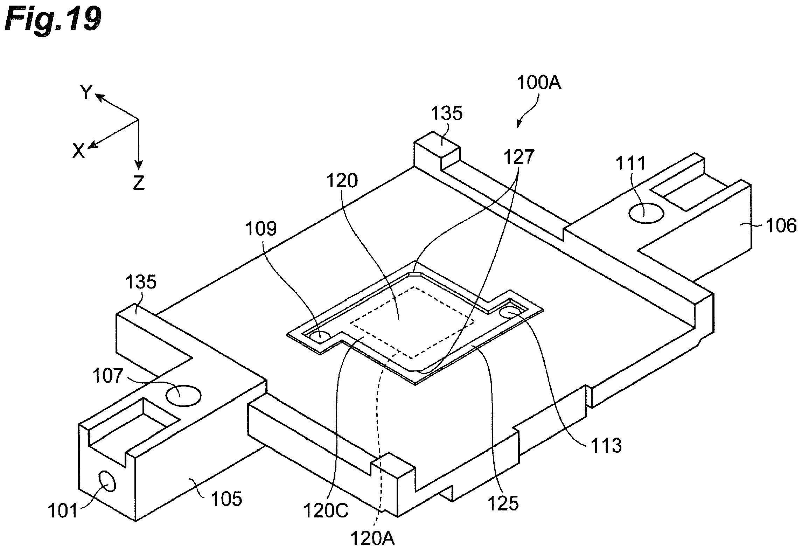

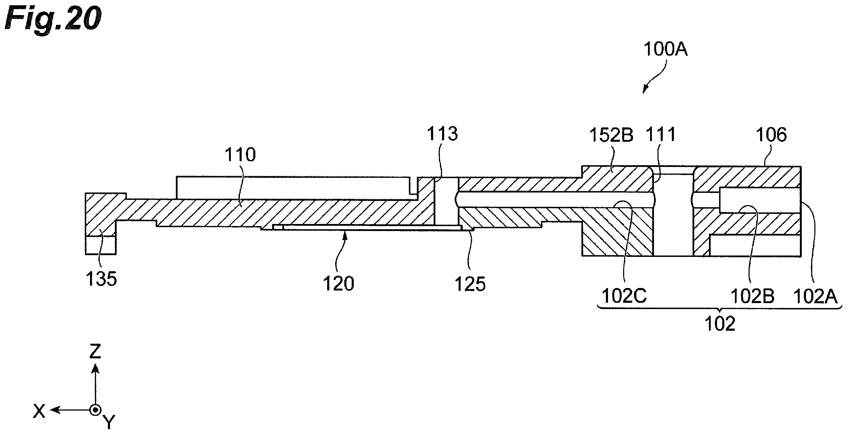

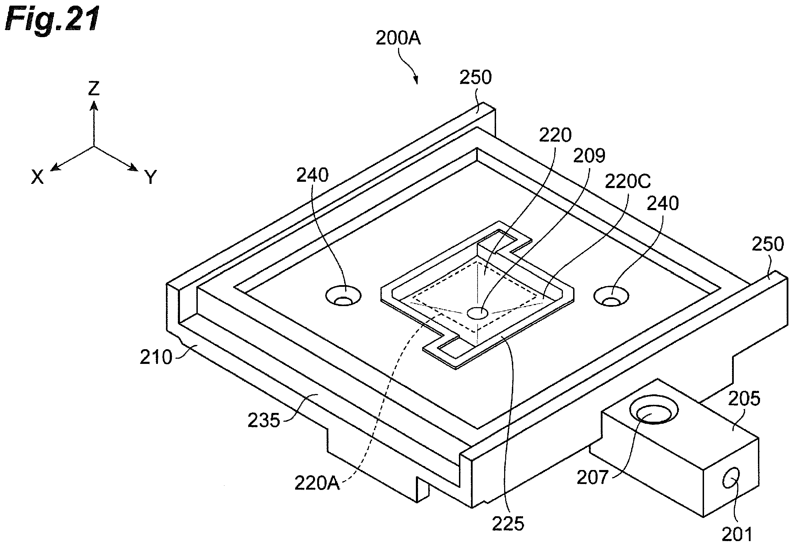

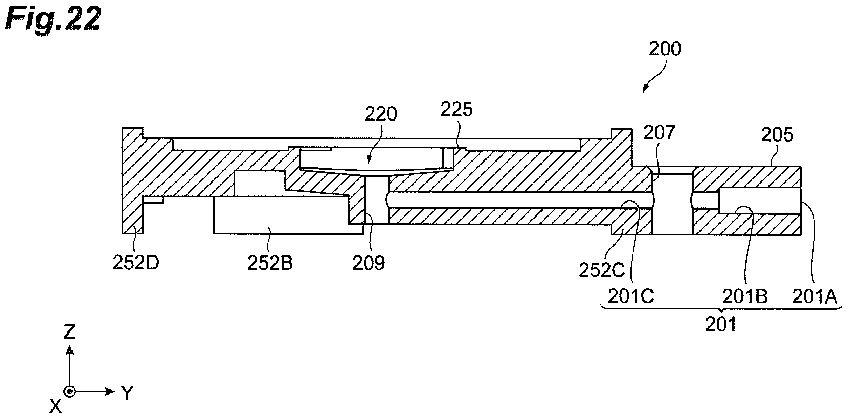

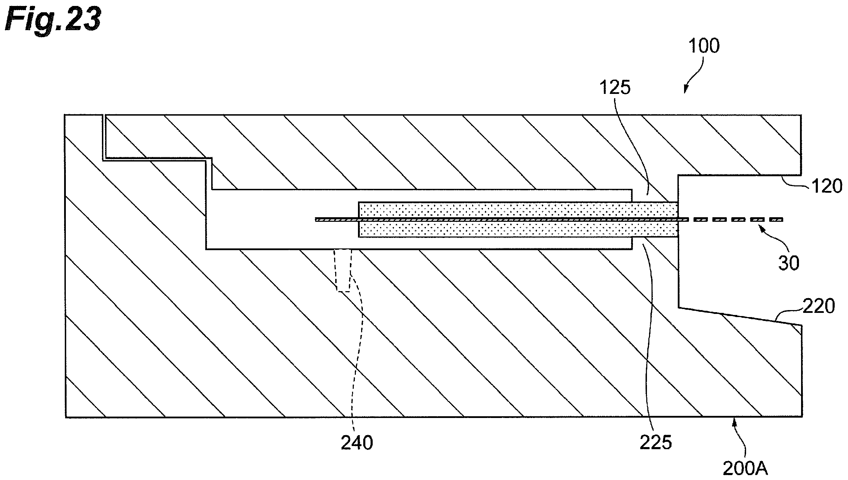

Inside shapes of the lid member and the housing member can also be adequately changed. In the cell capture device described in the embodiment, the configuration in which the first fitting part 130 of the lid member 100 and the second fitting part 230 of the housing member 200 are fitted is described. A configuration in which the first fitting part 130 and the second fitting part 230 are not provided, however, may be employed, for example. A lid member 100A and a housing member 200A configuring the cell capture device not providing the fitting parts will be described with reference to FIG. 19 to FIG. 23. FIG. 19 is a schematic perspective view of the lid member according to a modified example seen from below. FIG. 20 is a view illustrating an inner structure of the lid member according to a modified example and corresponding to the arrow view of the IV-IV cross-section in FIG. 2. FIG. 21 is a schematic perspective view of the housing member according to a modified example seen from above. FIG. 22 is a view illustrating an inner structure of the housing member according to the modified example and corresponding to the arrow view of the VII-VII cross-section in FIG. 5. FIG. 23 is a view corresponding to FIG. 15 schematically illustrating the cross-section after assembling the lid member, the cell capture filter, the sealing member, and the housing member according to the modified example.

As illustrated in FIG. 19 and FIG. 20, the lid member 100A according to the modified example is determined not to provide the first fitting part 130. As illustrated in FIG. 21 and FIG. 22, the housing member 200A according to the modified example is determined not to provide the second fitting part 230. When the cell capture device is assembled using such a lid member 100A and a housing member 200A, the fitting part is not disposed on the outer periphery side of the cell capture filter 30 as illustrated in FIG. 23. In the case of such a cell capture device, for example, the cell capture device preferably has a configuration in which the outer periphery side of the cell capture device can be more stably supported by changing the shape of the fastening member or other modifications. As described above, by the configuration in which the cell capture device does not have the fitting part, the cell capture device can be disassembled with the impact applied to the cell capture device being reduced when the cells captured on the cell capture filter 30 are taken out after capturing the cells. As a result, the captured cells can be effectively collected. As the shapes of the lid member and the housing member, both appearance shapes and internal shapes can be adequately changed.

In the above embodiment, the fastening members and the guide parts have one-to-one relation. The fastening members, however, can be mounted to one guide part. The shape of the fastening member is not limited to the above embodiment and can be adequately modified.

Including the fastening member, the method for fastening or fixing the lid member, the housing member, and the filter in the cell capture device is not limited to the above embodiment and other aspects may be employed. Examples of the other aspects include a method for fixing the lid member and the housing member after sandwiching the filter member by adhesive or welding.

### Reference Signs List

1... Cell Capture Device, 1A... Cell Capture Apparatus, 10... Chassis, 30... Cell Capture Filter, 36... Alignment Hole, 40... Sealing Member (Gasket), 60... Fastening Member, 100, 100A... Lid Member, 101, 102... Introduction Channel, 120... Introduction Region, 125... First Protrusion Part, 130... First Fitting Part, 200, 200A... Housing Member, 201... Discharge Channel, 220... Discharge Region, 225... Second Protrusion Part, 230... Second Fitting Part, 240... Bottomed Hole (Hole Part).

## Claims

1. A cell capture device comprising:
a chassis including a lid member having an introduction channel configured to introduce a test liquid into an inside, and a housing member having a discharge channel configured to discharge the test liquid to an outside; and
a cell capture filter disposed on a channel inside the chassis between the introduction channel and the discharge channel, including a filter region therein provided with a through-hole in a thickness direction and configured to pass the test liquid, wherein
the lid member includes a looped first protrusion part provided at a position outside of the filter region and inside of an outer edge of the cell capture filter in an assembled state and protruding outward from a surface of a side to which the cell capture filter is attached,
the housing member includes a looped second protrusion part provided at a position corresponding to the first protrusion part in the assembled state and located at a position corresponding to the first protrusion part to protrude, and
the channel inside the chassis is formed by locating the first protrusion part and the second protrusion part at the corresponding position to sandwich the cell capture filter in the assembled state.

2. The cell capture device according to claim 1, wherein
the lid member further includes a first fitting part provided at a position outside of and away from the first protrusion part and not overlapped with the cell capture filter in the assembled state, and fitted with the housing member,
the housing member further includes a second fitting part fitted with the first fitting part of the lid member in the assembled state, and
one of the first fitting part and the second fitting part forms a projection shape and the other forms a recess shape.

3. The cell capture device according to claim 1 or 2 further comprising a sealing member including an opening part in an inside, sandwiched between at least one of the first protrusion part and the second protrusion part and the cell capture filter, and having elasticity, wherein
the sealing member is located at all corresponding regions where the first protrusion part and the second protrusion part sandwich the cell capture filter, and
a size of the opening part is formed in accordance with a shape of the inner periphery of the first protruding part and the second protruding part.

4. The cell capture device according to any one of claims 1 to 3, wherein
the cell capture filter has a plurality of alignment holes at a position outside of the filter region and inside of the outer edge of the cell capture filter, and
at least one of the lid member and the housing member has hole parts provided at positions corresponding to the alignment holes.

5. A cell capture filter comprising a filter region therein provided with a through-hole in a thickness direction and configured to pass a test liquid, wherein
the cell capture filter is fixed without being folded, by a lid member having an introduction channel configured to introduce the test liquid into an inside and including a looped first protrusion part protruding outward from a surface of a side to which the cell capture filter is attached and a housing member having a discharge channel configured to discharge the test liquid to an outside and including a looped second protrusion part protruding outward from a surface of a side to which the cell capture filter is attached.

6. A cell capture filter configured to be fixed by a lid member having an introduction channel configured to introduce a test liquid into an inside and a housing member having a discharge channel configured to discharge the test liquid to an outside, wherein
the lid member includes a looped first protrusion part provided at a position outside of a filter region of the cell capture filter where through-holes configured to pass the test liquid are formed in a thickness direction and inside of an outer edge of the cell capture filter in a fixed state and protruding outward from a surface of a side to which the cell capture filter is attached,
the housing member includes a looped second protrusion part provided at a position corresponding to the first protrusion part in the fixed state and located at a position corresponding to the first protrusion part to protrude,
the first protrusion part and the second protrusion part are located at the corresponding position to sandwich the cell capture filter in the fixed state, and
the outer edge of the cell capture filter is located outside of a larger outer periphery of either the first protrusion part or the second protrusion part.

7. The cell capture filter according to claim 5 or 6, wherein
the lid member further includes a first fitting part provided at a position outside of and away from the first protrusion part and not overlapped with the cell capture filter in the fixed state, and fitted with the housing member,
the housing member further includes a second fitting part fitted with the first fitting part of the lid member in the fixed state,
one of the first fitting part and the second fitting part forms a projection shape and the other forms a recess shape, and
the outer edge of the cell capture filter is located inside of a smaller inner periphery of either the first fitting part or the second fitting part and outside of a larger outer periphery of either the first protrusion part or the second protrusion part.

8. The cell capture filter according to any one of claims 5 to 7, wherein
the cell capture filter has a plurality of alignment holes at a position outside of the filter region and inside of the outer edge of the cell capture filter.

9. A cell capture apparatus comprising:
the cell capture device according to any one of claims 1 to 4;
means for supplying a test liquid configured to supply the test liquid to the introduction channel of the cell capture device;
means for supplying a processing liquid configured to supply, to the introduction channel of the cell capture device, the processing liquid for processing the cells captured on the cell capture filter by passing through the cell capture filter; and
means for selecting configured to select a liquid to be supplied to the cell capture device from the test liquid and the processing liquid.

10. A method for manufacturing a cell capture device, the cell capture device comprising
a chassis having a lid member having an introduction channel configured to introduce a test liquid into an inside and a housing member having a discharge channel configured to discharge the test liquid to an outside, and
a cell capture filter disposed on a channel between the introduction channel and the discharge channel inside the chassis and including a filter region therein provided with a through-hole in a thickness direction and configured to pass the test liquid, the method for manufacturing the cell capture device comprising the step of:
forming the lid member including a looped first protrusion part provided at a position outside of the filter region and inside of an outer edge of the cell capture filter in an assembled state and protruding outward from a surface of a side to which the cell capture filter is attached;
forming the housing member including a looped second protrusion part provided at a position corresponding to the first protrusion part in the assembled state and located at a position corresponding to the first protrusion part to protrude; and
fixing the lid member, the housing member, and the cell capture filter by sandwiching the cell capture filter between the first protrusion part and the second protrusion part.

11. The method for manufacturing a cell capture device according to claim 10 comprising the steps of:
forming the lid member further including a first fitting part provided at a position outside of and away from the first protrusion part and not overlapped with the cell capture filter in the assembled state, and fitted with the housing member;
forming the housing member further including a second fitting part fitted with the first fitting part of the lid member in the assembled state;
forming a projection shape in one of the first fitting part and the second fitting part and forming a recess shape in the other; and
fitting the lid member, the housing member, and the cell capture filter so that the first protrusion part and the second protrusion part are located at the corresponding position to sandwich the cell capture filter and the first fitting part and the second fitting part are fitted with each other to fix the cell capture filter outside of the outer edge of the cell capture filter.
